# EUROPÄISCHE PATENTSCHRIFT

(11) **EP 3 541 263 B1**
(45) Veröffentlichungstag und Bekanntmachung des Hinweises auf die Patenterteilung: **05.04.2023**
(21) Anmeldenummer: 17832044.6
(22) Anmeldetag: 17.11.2017
(51) Int. Cl.: A61B 1/00, A61B 1/015, A61B 1/07, A61B 5/00, A61B 1/24, A61C 9/00, G02B 23/16, G02B 21/10

(54) **SCHUTZKAPPE FÜR EINE BILDGEBENDE VORRICHTUNG**
PROTECTIVE CAP FOR AN IMAGING DEVICE
COUVERCLE DE PROTECTION POUR UN DISPOSITIF DE PRISE D'IMAGES

(30) Priorität: 17.11.2016 DE 102016122102
(43) Veröffentlichungstag der Anmeldung: 25.09.2019
(73) Patentinhaber: Westfälische Wilhelms-Universität Münster, 48149 Münster (DE)
(72) Erfinder: HESSLER, Michael, 48149 Münster (DE); ARNEMANN, Philip-Helge, 48149 Münster (DE); ERTMER, Christian, 48149 Münster (DE)
(74) Vertreter: Schiweck Weinzierl Koch Patentanwälte Partnerschaft mbB
(86) Internationale Anmeldenummer: PCT/EP2017/079569
(87) Internationale Veröffentlichungsnummer: WO 2018/091645

(56) Entgegenhaltungen:
- EP-A1- 2 515 153
- EP-A2- 2 449 956
- WO-A1-2016/047191
- WO-A1-2016/147470
- WO-A1-2016/168764
- JP-A- 2008 272 168
- JP-A- 2009 279 159
- US-A- 5 997 164
- US-A1- 2003 009 085
- US-A1- 2006 184 037

## Beschreibung

Bei der vorliegenden Vorrichtung handelt es sich um eine Schutzkappe für eine bildgebende Vorrichtung, insbesondere um eine Schutzkappe für eine Kamera zur Beobachtung einer Mikrozirkulation, beispielsweise im Mundraum eines Patienten.

Die lebensbedrohlichen Krankheiten intensivmedizinischer Patienten sind häufig begleitet von Änderungen in der Mikrozirkulation (auch mikrovaskuläre Perfusion genannt). Die Mikrozirkulation wird zunehmend als kritische Determinante der Organfunktion intensivmedizinischer Krankheitsbilder angesehen. In den letzten Jahren sind daher neue Methoden für die Visualisierung der Mikrozirkulation entwickelt worden, wie zum Beispiel das sidestream darkfield imaging (abgekürzt SDF, auf Deutsch: Seitenstrom-Dunkelfeld-Bildgebung) oder incident darkfield illumination imaging (abgekürzt IDF, auf Deutsch Auflicht-Dunkelfeld-Bildgebung). Durch diese Methoden ist mittels entsprechender Untersuchungsgeräte eine direkte Beobachtung der Mikrozirkulation am Patientenbett möglich. Die Untersuchung der Mikrozirkulation erfolgt zurzeit vor allem im Rahmen der universitären Medizin. Die direkte Untersuchung der Mikrozirkulation ist ein wertvolles diagnostisches Werkzeug und kann dabei helfen, das Krankheitsstadium eines intensivmedizinischen Patienten besser einzuschätzen. Es kann angenommen werden, dass in den nächsten Jahren Therapieentscheidungen auf der Basis von mikrozirkulatorischen Parametern erfolgen werden.

In der Regel wird im Rahmen der oben genannten Methoden die Mikrozirkulation der Patienten durch Einführen einer kleinen stiftförmigen Kamera in den Mund untersucht, wobei die Kameraspitze unter die Zunge (sublingual) platziert wird. Die Kamera ist dabei von einer durchsichtigen keimarmen Schutzkappe bedeckt, welche aus hygienischen Gründen jeweils nach erfolgter Untersuchung eines Patienten von der Kamera gelöst und entsorgt wird. Zur Untersuchung eines nächsten Patienten wird auf die Kamera eine unbenutzte Schutzkappe aufgesetzt. In diesem Zusammenhang sei hier beispielhaft die CytoCam^{®} der Firma Braedius erwähnt. Dabei handelt es sich um eine Kamera zur Beobachtung der Mikrozirkulation der Firma Braedius, welche auf der Auflicht-Dunkelfeld-Bildgebung basiert und sich bestens zur direkten Visualisierung der sublingualen Mikrozirkulation eignet. Auch bei dieser Kamera kommen die beschrieben Einweg-Schutzkappen zum Einsatz.

Generell erfolgt die Beobachtung der sublingualen Mikrozirkulation, indem eine mit einer Schutzkappe geschützte Kamera an die sublinguale Schleimhaut angelegt bzw. in Kontakt mit dieser gebracht wird. Hierdurch können die unter der Schleimhautoberfläche liegenden Haargefäße (Kapillaren) dargestellt werden.

Ein wesentliches Problem bei der Untersuchung der Mikrozirkulation stellt der geringe intraluminale Druck in den Kapillaren dar. Beim Aufsetzten der Kamera auf die sublinguale Schleimhaut können die Kapillaren bereits durch den Druck auf die sublinguale Schleimhaut abgedrückt werden, welcher durch das Anlegen der Kamera erzeugt wird. Dies kann mitunter eine schlechte Perfusion der Mikrozirkulation suggerieren, obwohl eigentlich keine Störung der mikrovaskulären Perfusion vorliegt. Dieser Umstand wird auch mit dem Terminus Druckartefakt bezeichnet und kann letztendlich zu einer Falschdiagnose bezüglich des Gesundheitszustandes eines intensivmedizinischen Patienten und damit zu höchstwahrscheinlich für den Patienten nachteiligen Therapieentscheidungen führen.

Ein weiteres Problem bei der Untersuchung der Mikrozirkulation stellt das Vorhandensein von übermäßigem Speichel, Zelltrümmern (u.a. von roten Blutkörperchen) oder Luftblasen am Zungenboden dar. Das Vorliegen dieser Materialien im Untersuchungsbereich erschwert die Untersuchung der Mikrozirkulation erheblich und kann mitunter die Untersuchung der Mikrozirkulation sogar unmöglich machen.

Dokument US 2006/0184037 A1 offenbart eine Schutzkappe gemäß dem Oberbegriff von Anspruch 1.

Ziel der vorliegenden Erfindung ist es, die oben genannten Probleme bei der Untersuchung der Mikrozirkulation zu umgehen. Diese Probleme werden durch die Vorrichtung gemäß Anspruch 1 gelöst. Weitere bevorzugte Ausführungsformen sind in den Unteransprüchen beschrieben.

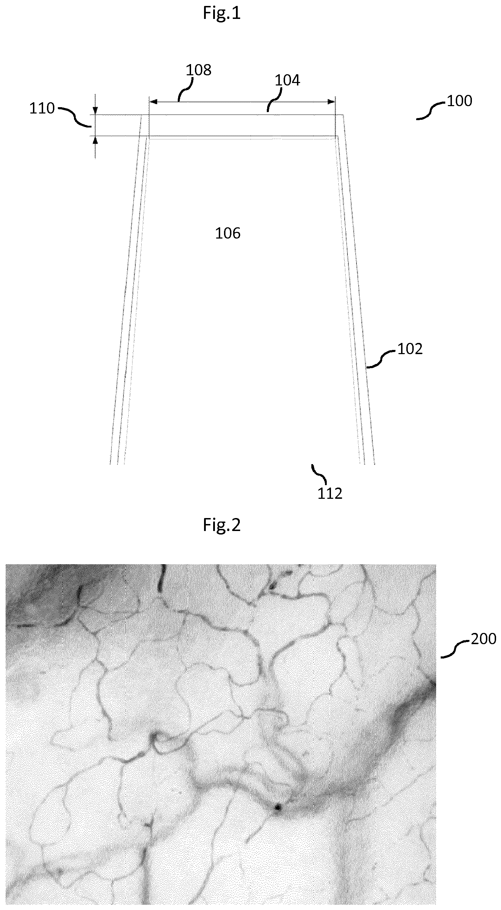

Bevor die Erfindung genauer erläutert wird, soll zunächst eine aus dem Stand der Technik bekannte Schutzkappe für bildgebende Vorrichtungen zur Untersuchung der Mikrozirkulation kurz beschrieben werden. Eine solche beispielhafte Schutzkappe ist schematisch in Figur 1 dargestellt und wird später im Detail beschrieben. Die Schutzkappen der derzeit erhältlichen Kameras zur Beobachtung der Mikrozirkulation bestehen aus einem durchsichtigen Kunststoff, durch den hindurch mittels der Kamera die Mikrozirkulation beobachtet werden kann. Bedingt durch die stiftförmige Form der Kamera sind gattungsgemäße Schutzkappen im Wesentlichen ebenfalls stiftförmig oder konisch geformt, so dass der stiftförmige Teil der Kamera, an dessen Ende sich die Optik befindet, in die Schutzkappe eingesetzt werden kann. Die gattungsgemäße Schutzkappe ist starr und aus einem ca. 1 mm dicken durchsichtigen Kunststoff gefertigt. Die Schutzkappe wird vor der Untersuchung über das stabförmige Ende der Kamera gesteckt und schließt am Kamerakörper dicht ab. Zur Beobachtung der Mikrozirkulation wird die Schutzkappe direkt auf die Schleimhaut aufgesetzt. Die Kontaktfläche der Schutzkappe mit der Schleimhaut besteht aus einer planen Fläche.

Ausgehend von dieser Situation liegt der Erfindung die Aufgabe zu Grunde, die Untersuchung der Mikrozirkulation zu verbessern, insbesondere das Auftreten von Druckartefakten zu unterbinden und im Weiteren die Abbildungsqualität zu verbessern, welche durch übermäßigen Speichel, Zelltrümmern und/oder Luftblasen, beeinträchtigt sein kann.

Bei der erfindungsgemäßen Vorrichtung handelt es sich um eine Schutzkappe für eine bildgebende Vorrichtung, welche einen hohlen Schutzkappenkörper mit einer Stirnfläche, einer Mantelfläche und einem offenen hinteren Ende aufweist, durch welches die bildgebende Vorrichtung in die Schutzkappe eingesetzt werden kann, und eine Vertiefung, welche in der Stirnfläche angeordnet ist. Die Schutzkappe weist ferner mindestens einen Kanal auf, welcher im vorderen Teil des Schutzkappenkörpers unter der Stirnfläche angeordnet ist, wobei der mindestens eine Kanal eine erste Öffnung aufweist, welche in der Seitenwand der Vertiefung angeordnet ist, und eine zweite Öffnung aufweist, welche bevorzugt in der Mantelfläche des Schutzkappenkörpers angeordnet ist.

Abweichend zu bisher bekannten Schutzkappen, welche wie eingangs erwähnt eine durchgehend plane Stirnseite aufweisen, ist die erfindungsgemäße Schutzkappe dahingehend modifiziert, dass sie eine Vertiefung (Aussparung) in der Stirnfläche aufweist. Diese Modifikation ermöglicht es, Druckartefakte im Untersuchungsbereich zu reduzieren oder sogar ganz zu vermeiden. Bei gattungsgemäßen Schutzkappen wird an Stellen, an denen die Schutzkappe auf die Schleimhaut aufgesetzt wird, Druck auf die betroffene Schleimhaut ausgeübt. Dieser Druck führt zur Kompression oberflächlicher Kapillarschlingen, wodurch der Fluss in den in dem komprimierten Bereich vorliegenden Kapillaren behindert wird und diskontinuierlich wird; es kommt zur Bildung von Druckartefakten. Bei der Schutzkappe gemäß verschiedenen Ausführungsbeispielen ist in der Stirnfläche des Schutzkappenkörpers, beispielsweise in der Mitte des Bereichs, welcher während der Untersuchung in Kontakt mit der Schleimhaut steht, eine Vertiefung eingearbeitet. Dadurch kann die in diesem Bereich erfolgende Untersuchung der Mikrozirkulation (z.B. durch Videoaufnahme der Mikrozirkulation) ohne Druckartefakte erfolgen.

Die erfindungsgemäße Schutzkappe kann eine längliche bzw. stiftförmige Form aufweisen, beispielsweise die Form eines stumpfen Kegels, wobei die Deckfläche der Stirnfläche entspricht und die Grundfläche dem offenen Ende des Schutzkappenkörpers entspricht. Die Form der Schutzkappe kann insgesamt an die Form des Teils der bildgebenden Vorrichtung angepasst sein, den sie abdecken soll. Bei der bildgebenden Vorrichtung kann es sich um eine beliebige Kamera handeln, die zur Beobachtung der Mikrozirkulation geeignet ist. Beispielsweise kann es sich dabei um die CytoCam^{®} der Firma Braedius handeln. Die erfindungsgemäße Schutzkappe kann aus einem beliebigen geeigneten Material hergestellt sein, etwa einem Kunststoff, und kann beispielsweise durch Sintern oder 3D-Druck gefertigt werden.

Die erfindungsgemäße Schutzkappe weist in der Vertiefung einen Boden auf. Mit anderen Worten handelt es sich bei der Vertiefung um eine gegen den im Inneren des Schutzkappenkörpers liegenden Hohlraum verschlossene Vertiefung oder Ausnehmung. Dabei schließt der Boden die Vertiefung gegenüber dem innenliegenden Hohlraum des Schutzkappenkörpers dicht (fluiddicht), so dass Fluide oder sonstiges Materie nicht durch die Vertiefung hindurch in den Hohlraum der Schutzkappe gelangen kann. Der innenliegende Hohlraum der Schutzkappe, in dem bei einer Untersuchung des Patienten eine Kamera eingesetzt ist, kann somit steril gehalten werden.

Der Boden der erfindungsgemäßen Schutzkappe weist ein transparentes Material auf, beispielsweise ein Deckglas. Der Boden der Vertiefung kann integraler Bestandteil der gesamten Schutzkappe sein. Alternativ kann der Boden der Vertiefung nicht integral mit der Vertiefung ausgebildet sein. Der Boden der Vertiefung kann auch aus einem anderen Material als dem der Schutzkappe ausgebildet sein. Insbesondere kann der Boden ein Deckglas oder ein anderes Plättchen aus einem lichtdurchlässigen Material aufweisen. Dabei kann das den Boden ausbildende Material beispielsweise durch das offene Ende des Hohlraums in diesen eingebracht werden und an dem unteren Rand der Vertiefung und/oder im Bereich der Rückseite der Stirnfläche befestigt werden, etwa durch einen entsprechend dafür vorgesehenen Click-Mechanismus.

Im Hinblick auf den Boden der Vertiefung kann dieser gemäß weiteren Ausführungsbeispielen der Schutzkappe ein optisches Element aufweisen. Der Boden der Vertiefung kann aus einem transparenten Material geformt sein und als ein optisches Element eingerichtet sein. Beispielsweise kann das optische Element ein optischer Filter, eine Linse, eine Blende oder eine beliebige Kombination daraus sein. Allgemein kann es sich bei dem optischen Element um ein beliebiges zweckdienliches optisches Element handeln, welches an der Gestaltung des Lichtpfades vom Gewebe des untersuchten Patienten zu der in der Schutzkappe vorliegenden Kamera mitwirkt. Um ein konkretes Ausführungsbeispiel zu nennen, kann der Boden der Vertiefung über elektrische Anschlüsse verfügen und aus abdunkelbarem Glas bestehen und damit als eine variable Blende eingerichtet sein. Zusätzlich kann ein optischer Filter vorgesehen sein, welcher vor oder hinter der Blende angeordnet ist. Im Rahmen dieser Anmeldung ist die Beziehung zwischen dem Boden der Vertiefung und dem optischen Element weit zu sehen. Das heißt, dass das optische Element (oder mehrere optische Elemente) als ein Modul (als Module) eingerichtet sein kann (können) und mit einem Element, welches den physischen Boden der Vertiefung ausbildet (also die Materialschicht, welche die Vertiefung gegenüber dem Hohlraum dicht abschließt) gekoppelt sein kann (können). Es kann jedoch auch sein, dass das optische Element unmittelbar in der Materialschicht integriert ist, die den Boden der Vertiefung bildet.

Gemäß weiteren Ausführungsbeispielen der Schutzkappe kann an der Innenseite des Schutzkappenkörpers im Bereich der Stirnfläche mindestens ein Halteelement angeordnet sein, welches zur lösbaren Befestigung des Bodens der Vertiefung eingerichtet ist. Unter dem Bereich der Stirnfläche der Schutzkappe kann die Stirnfläche der Schutzkappe sowie der daran anliegende Bereich der Schutzkappe gemeint sein, also etwa die vordere Spitze der Schutzkappe, beispielsweise etwa das vordere Fünftel der Schutzkappe. Anders ausgedrückt kann die erfindungsgemäße Schutzkappe so eingerichtet sein, dass der Boden der Vertiefung als ein austauschbares Modul eingerichtet ist und beispielsweise durch das offene hintere Ende der Schutzkappe eingesetzt bzw. ausgetauscht werden kann. Das Halteelement kann beispielsweise als eine Ringnut eingerichtet sein, welche in der Innenwand des Hohlraums angeordnet ist. Dabei kann die Schutzkappe aus zwei Teilen bestehen, die gegeneinander verschraubt oder aneinander gesteckt werden können, wobei ein erster (z.B. oberer) Teil der Ringnut in dem ersten (z.B. oberen) Teil der Schutzkappe bereitgestellt ist und ein zweiter (z.B. unterer) Teil der Ringnut in dem zweiten (z.B. unteren) Teil der Schutzkappe bereitgestellt ist. Der Boden der Vertiefung kann vor dem Verschrauben bzw. Zusammenstecken der beiden Schutzkappenteile miteinander zwischen diese beiden eingesetzt werden.

Gemäß weiteren Ausführungsbeispielen kann die Stirnfläche des Schutzkappenkörpers um die Vertiefung herum plan ausgebildet sein. Dabei kann die Ebene der Stirnfläche parallel zur Ebene des Bodens der Vertiefung angeordnet sein. Zusätzlich optional kann der Winkel, unter dem die Mantelfläche auf die Stirnfläche in jedem Punkt entlang des äußeren Randes der Stirnfläche trifft, in etwa 90° oder mehr betragen. Je größer dieser Winkel, umso ausgeprägter die Kegelnatur der Schutzkappe gegenüber einem Zylinder. In analoger Weise kann auch der Winkel zwischen der Stirnfläche und der Wandung der Vertiefung in etwa 90° oder mehr betragen. Anders ausgedrückt kann die Stirnfläche um bis zu 90° nach unten umknicken und so die Wandung der Vertiefung ausbilden, die bis zum Boden der Vertiefung führt. Der Übergang zwischen Mantelfläche und Stirnfläche kann eine scharfe Kante oder eine gerundete Kante bilden. Der Übergang zwischen Stirnfläche und Wandung der Vertiefung kann in analoger Weise eine scharfe Kante oder eine gerundete Kante bilden. Die Aussage, dass um die Vertiefung herum die Stirnfläche des Schutzkappenkörpers plan ausgebildet ist kann, kann damit den Großteil der Stirnfläche betreffen, wobei der in der Stirnfläche liegende Randbereich der Vertiefung und/oder der Bereich des äußeren Randes der Stirnfläche davon ausgenommen sein können.

Gemäß weiteren Ausführungsbeispielen kann die Stirnfläche des Schutzkappenkörpers als eine radial zur Vertiefung hin ansteigende oder abfallende Oberfläche ausgebildet sein. Das heißt, dass der Außenrand der Stirnfläche, an dem die Stirnfläche in die Mantelfläche übergeht, in einer Querschnittsseitenansicht betrachtet tiefer oder höher liegen kann. Die ansteigende oder abfallende Eigenschaft der Stirnfläche muss nicht notwendigerweise strikt für jeden Bereich/Abschnitt der Stirnfläche gelten sondern ist als eine Aussage bezüglich des durchschnittlichen Verhaltens der Stirnfläche zu sehen. So kann es etwa sein, dass die Stirnfläche stufenartig oder anderweitig segmentiert zwar gesamt gesehen zur Öffnung der Vertiefung (Vertiefungsöffnung) hin ansteigt oder abfällt, aber in einigen Abschnitten plan verläuft (also weder ansteigend noch abfallend) oder sogar die zum Gesamtverhalten entgegengesetzte Steigung aufweist. Mit einer Ausführungsform der Schutzkappe mit einer radial zur Vertiefung hin abfallenden Stirnfläche und einem abgerundeten Rand an der Vertiefungsöffnung können Druckartefakte weiträumig um die unmittelbar untersuchte Gewebestelle vermieden werden. Zugleich jedoch kann ein am Gewebe anliegender Außenrand der Stirnfläche dafür sorgen, dass kein Streulicht von außen in die Vertiefung und damit letztendlich zur bildgebenden Vorrichtung gelangen kann.

Gemäß weiteren Ausführungsbeispielen kann die Stirnfläche des Schutzkappenkörpers zumindest in einem Bereich um die Vertiefung herum strukturiert sein. Der Bereich kann beliebig geformt sein, etwa konzentrisch oder elliptisch, und er kann symmetrisch oder asymmetrisch um die Vertiefungsöffnung angeordnet sein. Der Bereich kann sich auch über die gesamte Stirnfläche ausdehnen. Bei der Strukturierung kann es sich um eine beliebige zweckdienliche Strukturierung handeln, wie etwa eine genoppte oder geriffelte Strukturierung. Die Strukturierung kann funktionale Materialien aufweisen, d.h. Materialien, die z.B. eine verbesserte Griffigkeit gegenüber dem untersuchten Gewebe aufweisen (z.B. Mini-Saugglocken oder Mini-Saugnäpfe) oder spezielle optische (z.B. reflektierende) Eigenschaften haben. Die Strukturierung kann ebenfalls dahingehend funktional sein, als dass sie den Abtransport von übermäßigem Speichel, Zelltrümmern oder Luftblasen unterstützt. Beispielsweise kann gemäß weiteren Ausführungsbeispielen der Schutzkappe in der Stirnfläche des Schutzkappenkörpers mindestens eine Furche angeordnet sein, welche sich zwischen der Vertiefung und dem äußeren Rand der Stirnfläche erstreckt. Die mindestens eine Furche kann als Transportkanal verwendet werden, um beispielsweise das eben erwähnte unerwünschte Material aus dem Untersuchungs-/Beobachtungsbereich, welcher im Wesentlichen der Vertiefungsöffnung entspricht, abzutransportieren.

Gemäß der Erfindung weist die Schutzkappe mindestens einen Kanal auf, welcher im vorderen Teil des Schutzkappenkörpers unter der Stirnfläche verläuft. Der erste Kanal weist eine erste Öffnung auf, welche in der Seitenwand der Vertiefung oder in der Stirnfläche mündet oder angeordnet ist. Zusätzlich weist der Kanal teine zweite Öffnung auf, welche in der Mantelfläche des Schutzkappenkörpers angeordnet ist. Der Kanal kann zum einen die Rolle der oben erwähnten Furche übernehmen und zum Abtransport von unerwünschtem Material aus dem Untersuchungs-/Beobachtungsbereich dienen. Durch die erste Öffnung, welche in der Wandung der Vertiefung münden kann, kann das Material in den Kanal eintreten. Um das unerwünschte Material aktiv abzusaugen, kann dazu an der zweiten Öffnung des Kanals eine Leitung angeschlossen sein, mittels welcher in dem Kanal ein Unterdruck erzeugt werden kann. Andererseits kann der mindestens eine Kanal auch als Zuleitung fungieren und beispielsweise zum Einbringen von Luft oder flüssigen, bevorzugt transparenten Medien in die Vertiefung verwendet werden. Hierbei erfüllt der mindestens eine Kanal die Rolle eines Spülkanals. Durch Einbringen einer optisch aktiven Substanz (z.B. eines Immersionsöls) in die Vertiefung kann die erzielbare Auflösung des während einer Patientenuntersuchung aufgenommenen Bildes gesteigert werden. Andererseits kann der mindestens eine Kanal mindestens eine Öffnung aufweisen, welche in der Stirnfläche mündet, beispielsweise in einem Bereich der Stirnfläche um die Vertiefung herum. Durch Bereitstellen eines Unterdrucks in dem mindestens einen Kanal kann dieser Unterdruck an der mindestens einen Öffnung dafür sorgen, dass an der Öffnung anliegendes Gewebe angesaugt und somit fixiert wird. Der mindestens eine Kanal kann also vielfältige Funktionen erfüllen, wobei diverse Parameter wie seine Bemaßung, sein Verlauf, die Anzahl und Lageort der Öffnungen an die beabsichtigte Verwendung angepasst werden können. Insgesamt sind natürlich auch Ausführungsbeispiele der Schutzkappe mit mehreren Kanälen im Rahmen der Erfindung enthalten, wobei die eben beschriebenen Arten der Kanäle in einer beliebigen Kombination in ein und derselben Schutzkappe vorkommen können. So kann zum Beispiel unter den im vorderen Teil der Schutzkappe verlaufenden Kanälen mindestens ein Kanal als Spülkanal und mindestens ein weiterer Kanal als Zuleitung dienen. Generell kann es von Vorteil sein, wenn die Öffnung des als Leitung zum Abtransport von unerwünschtem Material fungierenden Kanals der Öffnung des als Spülkanal fungierenden Kanals im Wesentlichen gegenüber angeordnet ist, beispielsweise diametral gegenüber angeordnet ist.

Bei der Verwendung des Kanals als Zuleitung kann als flüssiges transparentes Medium eine viskose Flüssigkeit in die Vertiefung eingebracht werden. Die Einleitung des flüssigen Mediums in die Vertiefung kann kontinuierlich oder bolusweise erfolgen, beispielsweise mittels einer Spritze oder Pumpe, die an den mindestens einen Kanal angeschlossen werden kann. Mindestens ein weiter Kanal oder mindestens eine oben erwähnte Furche kann verwendet werden zum Abtransport von Material aus der Vertiefung, wobei es sich bei dem Material um Detritus und/oder Speichel bzw. das vom Speichel verdünnte flüssige Medium, etwa das viskose Gel, handeln kann. Dazu kann an die außenliegende Öffnung des Abtransportkanals eine Spritze oder eine Pumpe angekoppelt werden und der Absaugvorgang kann kontinuierlich oder intermittierend erfolgen. Bei Verwendung eines Abtransportkanals kann dessen Durchmesser größer ausfallen als der Durchmesser des für die Einleitung des flüssigen Mediums verwendeten Kanals, um das Risiko einer Verstopfung bzw. Blockierung des Abtransportkanals zu minimieren.

Das Einleiten eines Mediums in die Vertiefung kann zudem vorteilhaft sein, insbesondere eines viskosen Gels, da es einen leichten Gegendruck auf das beobachtete Gewebe (d.h. das Gewebe im Bereich der Vertiefung) aufbauen kann. Dadurch kann verhindert werden, dass das Gewebe im Beobachtungsbereich (d.h. im Bereich der Vertiefung der Schutzkappe) nicht in die Vertiefung "fällt" bzw. expandiert und damit im Randbereich der Vertiefung Kapillaren in dem Gewebe abgedrückt werden. Mit anderen Worten kann das in die Vertiefung eingebrachte flüssige Medium dafür sorgen, dass das im Bereich der Vertiefung angeordnete Gewebe sich nur leicht in die Richtung der Vertiefung wölbt. Um zu verhindern, dass das in die Vertiefung eingeleitete Medium keinen zu großen Gegendruck aufbaut, können in der Stirnfläche der Kappe, wie oben erwähnt, Nuten bzw. Furchen und/oder weitere Kanäle angeordnet sein, die von der Vertiefung radial nach außen zum Mantelbereich der Schutzkappe verlaufen und so ein Abließen des flüssigen Medium, etwa des viskosen Gels, erlauben. Die Dimension der für den Druckabbau bereitgestellten Nuten und/oder Kanäle kann an das flüssige Medium angepasst werden, etwa an seine Viskosität, und so dessen kontrolliertes Abließen mit einer vorbestimmten Rate ermöglichen.

Das Einleiten des flüssigen Mediums in die Vertiefung während einer erfolgenden Untersuchung kann zudem für eine aktive Befeuchtung der untersuchten Schleimhaut sorgen, welche insbesondere bei Intensivpatienten häufig sehr trocken ist. Zugleich kann das flüssige und bevorzugt viskose Medium einen Gleitfilm auf der Schleimhaut ausbilden und so die Gleiteigenschaft der Schutzkappe auf der Schleimhaut verbessern, was Verletzungen der Schleimhaut vorbeugen kann. Zusätzlich kann das flüssige Medium in der Vertiefung eine ansonsten an der Grenzfläche zwischen Luft und feuchter Schleimhaut auftretende Reflexionen vermindern bzw. verhindern, welche die Beobachtung der Schleimhaut durch die Schutzkappe hindurch stören kann.

Bei dem verwendeten flüssigen viskosen Medium kann es sich beispielsweise um aus der Augenheilkunde bekannte hochviskose Flüssigkeiten auf Grundlage von Hyaluronaten (z.B. MICROVISC^{®} Natrimhyaluronat 1%) oder Hydroxypropylmethylcellulose (z.B. POLYVISC^{®} 2%) handeln.

Gemäß weiteren Ausführungsbeispielen der Schutzkappe kann die Seitenwand der Vertiefung und/oder die Stirnfläche der Schutzkappe ein lichtundurchlässiges (transpatentes) Material aufweisen. Mit der Seitenwand der Vertiefung ist generell die von der Stirnfläche aus im Wesentlichen nach unten verlaufende Wandung gemeint, die sich bis zum Boden der Vertiefung erstreckt. Das Bereitstellen von lichtdurchlässigem Material in den genannten Bereichen kann gewährleisten, dass maximal viel Licht aus dem untersuchten Bereich des Gewebes zum Objektiv der Kamera gelangt. Um zu verhindern, dass hingegen kein störendes Streulicht von der Umgebung des untersuchten Gewebebereiches zum Objektiv der Kamera gelangt, kann die Mantelfläche aus einem lichtundurchlässigen Material gefertigt sein.

Gemäß weiteren Ausführungsbeispielen kann in einem weiteren Kanal ein Lichtwellenleiter angeordnet sein. Dieser Kanal kann damit zur Lichtführung verwendet werden, wobei das Licht aus einer externen Lichtquelle in den Lichtwellenleiter eingekoppelt werden kann und mittels des Lichtwellenleiters an die gewünschte Stelle geführt werden kann. Über entsprechend positionierte Öffnungen kann der Lichtwellenleiter Licht an gewünschte Stellen der Schutzkappe geführt werden, um das untersuchte Gewebe zusätzlich auszuleuchten. Besonders bevorzugt kann hier Licht mit einer Wellenlänge (oder mehreren Wellenlängen) ins beobachtete/untersuchte Gewebe eingebracht werden, welche von der Wellenlänge, die von der Kamera verwendet wird, unterschiedlich ist. Das kann besonders nützlich sein, wenn man im beobachteten Gewebe nur bestimmte ausgewählte Strukturen mittels resonanter Anregung sichtbar machen will. Zur gezielten Sichtbarmachung der Mikrozirkulation, welche auf den Durchfluss von Blut und damit Hämoglobin zurückgeführt werden kann, kann grünes Licht mit einer Wellenlänge von 525 nm in das beobachtete Gewebe eingestrahlt werden.

Weitere Vorteile und Merkmale der Erfindung ergeben sich aus den nachfolgenden beispielhaften Erläuterungen mit Bezug auf die Figuren. Die in den Figuren gezeigten und/oder nachfolgend erläuterten Merkmale können, unabhängig von konkreten Merkmalskombinationen, allgemeine Merkmale der Erfindung sein, die losgelöst von der hier erfolgten Darstellung auf andere Ausführungsbeispiele der Erfindung übertragbar sind.
Figur 1 zeigt eine gewöhnliche Schutzkappe für eine Kamera zur Untersuchung der Mikrozirkulation.
Figur 2 zeigt eine Momentaufnahme, auf welcher ein Abbild der sublingualen Mikrozirkulation zu sehen ist.
Figur 3 zeigt eine Möglichkeit der Visualisierung der Mikrozirkulation.
Figur 4 zeigt eine schematische Seitenansicht einer Grundform der erfindungsgemäßen Schutzkappe.
Figur 5 zeigt ein weiteres Ausführungsbeispiel der erfindungsgemäßen Schutzkappe.
Figur 6 veranschaulicht den vorteilhaften Effekt der Vertiefung der erfindungsgemäßen Schutzkappe bei der Untersuchung der Mikrozirkulation.
Figur 7 zeigt eine weitere Ausführungsform der Schutzkappe.
Figuren 8A-8D zeigen 3D-Dartellung der Schutzkappe gemäß weiteren Ausführungsbeispielen.

In den nachfolgend beschriebenen Figuren werden gleiche Elemente, die in verschiedenen Figuren auftauchen, mit den gleichen Bezugszeichen versehen.

In **Figur 1** ist eine gewöhnliche Schutzkappe 100 für eine Kamera zur Untersuchung der Mikrozirkulation gezeigt. Die Kamera, beispielweise die CytoCam^{®} der Firma Braedius, wird in den hohlen Innenraum 106 der Schutzkappe 100 eingesetzt und kann so während einer Untersuchung der Mikrozirkulation im Mundraum eines Patienten steril gehalten werden. Die gewöhnliche Schutzkappe 100 ist aus einem Kunststoff gefertigt und ist konisch geformt. Das hintere Ende 112 der Schutzkappe, durch das die Kamera in das Innere 106 der Schutzkappe eingesetzt werden kann, ist offen. An die Mantelfläche 102 schließt sich eine Frontseite 104 an, welche aus einem transparenten Material gefertigt ist, so dass die Mikrozirkulation mittels der Kamera durch die Frontseite 104 beobachtet werden kann. Die Frontseite 104 kann in der Draufsicht rund ausgebildet sein und im Wesentlichen einen Durchmesser 108 von ca. 9 mm aufweisen. Die Dicke 110 der die Frontseite 104 ausbildenden Schicht beträgt ca. 1 mm.

**Figur 2** ist eine Momentaufnahme 200, welche ein Abbild der sublingualen Mikrozirkulation dargestellt, wie es mit einer dafür eingerichteten Kamera (samt aufgesetzter Schutzkappe) aufgenommen werden kann. Das Abbild wurde unter Ausleuchtung des Gewebes mit grünem Licht bei 525 nm erzeugt, welches vom Hämoglobin nahezu vollständig absorbiert wird. Daher erscheinen in dieser Darstellung blutdurchflossene Strukturen schwarz.

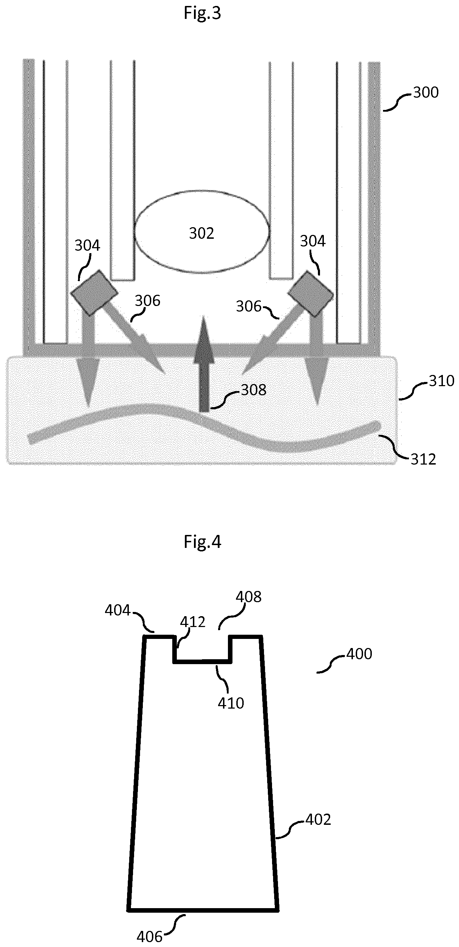

Die Mikrozirkulation kann beispielsweise mittels Auflicht-Dunkelfeld-Bildgebung sichtbar gemacht werden, die im Folgenden anhand der **Figur 3** erläutert werden soll (Figur stammt aus van Elteren HA; Journal of Clinical Monitoring and Computing; 2015 Oktober; 29(5):543-8). Dazu kann eine entsprechend eingerichtete Kamera verwendet werden, welche in Figur 3 durch eine Linse 302 repräsentiert ist. Wie dargestellt ist zudem die stiftförmige Kamera in von einer Schutzkappe 300 umgeben. Bei der Schutzkappe 300 kann es sich um die in Figur 1 dargestellte Schutzkappe handeln. Die Kamera ist mit zusätzlichen Lichtquellen 304 ausgestattet, z.B. mit grünen LEDs, welche das Gewebe 310 ausleuchten, mit dem die Schutzkappe 300 in Kontakt gebracht wird. Bei Verwendung von grünem Licht erscheint Hämoglobin schwarz, da es um 525 nm ein breites Absorptionsspektrum aufweist. Wie bereits erläutert, stellen Druckartefakte ein wesentliches Problem bei der Begutachtung der Mikrozirkulation dar. Durch Auflegen der Schutzkappe 300 auf das zu untersuchende Gewebe 310 können die feinen oberflächlich vorliegenden Kapillaren 312 abgedrückt werden, was zu einer verfälschten Darstellung der Mikrozirkulation führen kann und damit letzten Endes zu einer falschen Diagnose.

In **Figur 4** ist eine schematische Seitenansicht einer Grundform der erfindungsgemäßen Schutzkappe 400 dargestellt. Wie die aus dem Stand der Technik bekannte und in Figur 1 dargestellte gewöhnliche Schutzkappe 100 weist auch die Schutzkappe 400 gemäß verschiedenen Ausführungsbeispielen eine längliche Form auf, die beispielsweise einem Zylinder oder Kegelstumpf entsprechen kann. Der Schutzkappenkörper weist im Wesentlichen eine Mantelfläche 402 und eine Stirnfläche 404 auf sowie ein offenes hinteres Ende 406. Im Unterschied zu der bekannten gewöhnlichen Schutzkappe weist jedoch die erfindungsgemäße Schutzkappe 400 eine Vertiefung 408 in der Stirnfläche 404 auf. Die Vertiefung 408 kann dabei beispielsweise zentriert in der (in Draufsicht betrachtet) kreisrunden Stirnfläche 404 vorliegen. Die Vertiefung 408 weist eine Seitenwand bzw. eine Wandung 412 auf, welche sich bis zum Boden 410 der Vertiefung 408 erstreckt. Die geometrische Ausgestaltung der Stirnfläche 404 samt der Vertiefung 408 kann vielfältig ausfallen. In der dargestellten beispielhaften Darstellung in Figur 4 sind die Ecken beim Übergang der Mantelfläche 402 zur Stirnfläche 404 sowie beim Übergang der Stirnfläche 404 zur Wandung 412 der Vertiefung 408 spitz bzw. eckig. Jeder dieser Übergänge kann jedoch auch gerundet sein (unabhängig von dem jeweils anderen Übergang). Des Weiteren können die Winkel an den Übergängen zwischen den genannten Flächen, die in Figur 4 in erster Näherung bei ca. 90° liegen, anders eingerichtet werden. Insbesondere muss die Stirnfläche 404 nicht plan sein, sondern kann eine Strukturierung aufweisen oder zum Rand der Vertiefung 408 hin ansteigen oder abfallen. Je nach Ausführungsform der Schutzkappe 400 kann mindestens ein Teil des Bodens 410 und optional mindestens ein Teil der Wandung 412 und mindestens ein Teil der Stirnfläche 404 aus einem transparenten Material gefertigt sein. Dadurch wird gewährleistet, dass die im Inneren der Schutzkappe 400 befindliche Kamera das beobachtete Gewebe abbilden kann und aber auch, dass bei Bedarf zusätzliches Licht aus der Schutzkappe 400 in das Gewebe eingestrahlt werden kann. Schließlich kann auch das Verhältnis zwischen der Größe der Öffnung der Vertiefung 408 und der Größe der Stirnfläche 404 nach Bedarf variiert werden. Es ist zu betonen, dass die in Figur 4 gezeigte Querschnittsansicht leidglich eine schematische Darstellung der erfindungsgemäßen Schutzkappe ist und die Relationen der einzelnen Bestandteile zueinander vielfältig angepasst werden können. Alle Ausführungsformen haben jedoch die Gemeinsamkeit, dass in der Stirnfläche 404 eine Vertiefung 408 angeordnet ist, wodurch während einer Untersuchung der Mikrozirkulation der mittels der Kamera beobachtete Bereich des Gewebes nicht in Kontakt mit der Schutzkappe 400 steht, sondern wegen der Vertiefung 408 druckfrei ist.

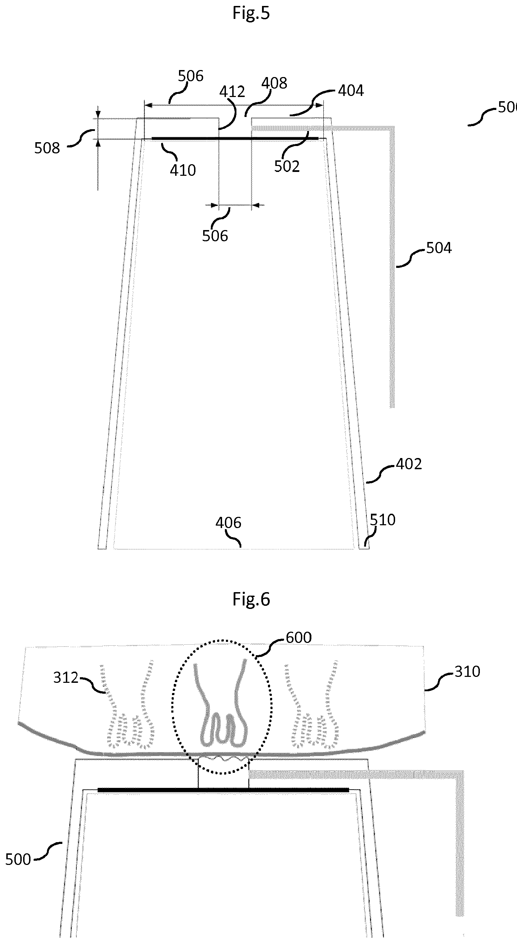

Eine weitere Schutzkappe 500 gemäß verschiedenen Ausführungsbeispielen ist in **Figur 5** gezeigt. Elemente, die bereits mit Bezug auf Figur 4 erläutert worden sind, werden nicht erneut beschrieben. Bei dem in Figur 5 gezeigten Ausführungsbeispiel der Schutzkappe 500 ist der Boden 410 der Vertiefung als ein an der Rückseite der Stirnfläche 404 anliegende Plättchen aus transparentem Material ausgebildet. Hierbei kann das Materialplättchen an der Rückseite der Stirnfläche permanent befestigt sein oder mittels entsprechender Haltemechanismen lösbar (im Sinne von austauschbar) fixiert sein. Der Austausch des Bodenplättchens 410 kann dabei über das offene hintere Ende 406 der Schutzkappe 500 erfolgen. In der gezeigten schematischen Querschnittsansicht sind ferner Dimensionen eingezeichnet zur Veranschaulichung eines konkreten Anwendungsbeispiels, wobei die Dicke 508 der Stirnfläche 404 und der Durchmesser 506 der Stirnfläche 404 insbesondere wegen der Kompatibilität zu den am Markt verfügbaren Kameras dieselben Maße haben können wie die entsprechenden Maße bei gewöhnlichen Schutzkappen, d.h. ca. 1 mm und ca. 9 mm. Der Durchmesser 506 des Bodens der Vertiefung 408 kann beispielsweise 3 mm betragen. Es sollte klar sein, dass diese Parameter je nach Bedarf angepasst werden können, ohne vom Wesen der Erfindung abzuweichen.

In der Darstellung in Figur 5 ist ein weiteres Element gezeigt, welches unabhängig von allen anderen frei anpassbaren Eigenschaften der erfindungsgemäßen Kappe bereitgestellt werden kann, nämlich ein Kanal 502. Obgleich nur ein Kanal 502 dargestellt ist, ist er stellvertretend für eine Anzahl an Kanälen zu sehen, die in der Materialschicht unter der Stirnfläche 404 angeordnet sein können. Der Kanal 502 weist eine erste Öffnung auf, welche in der Wandung 412 der Vertiefung 408 mündet. Der Kanal 502 weist zudem eine zweite Öffnung auf, welche in der Mantelfläche 402 angeordnet ist. Im gezeigten Ausführungsbeispiel ist zudem eine Leitung 504 an die zweite Öffnung des Kanals 502 angeschlossen. Wie bereits beschrieben, kann der (mindestens eine) Kanal 502 zur Erzeugung eines Unterdrucks in der Vertiefung 408, zum Einleiten eines Fluides in die Vertiefung 408 oder zum Absaugen von unerwünschtem Material aus der Vertiefung 408 verwendet werden. Die Öffnungen eines Kanals müssen nicht in einer 1:1 Beziehung zum Kanal 502 stehen. Das heißt, dass ein Kanal sich beispielsweise Verästeln kann und die Öffnung in der Mantelfläche 402 mit mehreren Öffnungen in der Wandung 412 der Vertiefung 408 in Kontakt stehen kann. Ebenso, sofern es erforderlich ist, kann der Kanal 502 (zusätzlich oder exklusiv) mindestens eine Öffnung aufweisen, welche in der Stirnfläche 404 angeordnet ist. Das kann beispielsweise dann von Vorteil sein, wenn mittels Unterdruck das um die Vertiefung 408 angeordnete Gewebe während einer Untersuchung angesaugt und/oder fixiert werden soll. Schließlich ist auch die Lage der zweiten Öffnung in der Mantelfläche 402 beliebig. So kann sie abweichend von der in Figur 5 gezeigten Lage weiter zum offenen hinteren Ende 406 der Schutzkappe 500 angeordnet sein. Die zweite Öffnung kann aber auch im Rand 510 der Mantelfläche 402 angeordnet sein, wobei der Kanal 502 dann im Mantel der Schutzkappe 500 integriert sein kann. Eine solche Anordnung kann den Vorteil haben, dass die angeschlossene Leitung 502 kürzer ausfallen kann, ihr Loslösen von der Öffnung unwahrscheinlicher ist und, dass so die entsprechende Schutzkappe ohne seitlich abstehende Leitung 504 kompakter ausfällt und damit in kleine Lücken und Ecken im untersuchten Gewebe vordringen kann.

Die Wirkung, welche die Vertiefung der erfindungsgemäßen Schutzkappe bei der Untersuchung der Mikrozirkulation im Gewebe hat, soll anhand der **Figur 6** verdeutlicht werden. In der Figur ist zum einen schematisch eine Gewebsschicht 310 samt darin vorliegenden Kapillaren 312 bzw. Kapillarschlingen dargestellt sowie eine in Kontakt mit dieser Gewebsschicht 310 stehende Schutzkappe 500, in der sich eine Kamera befindet (in Figur 6 nicht explizit dargestellt). In der Darstellung sieht man, dass der Bereich des Gewebes 310, auf welchem die Schutzkappe 500 mit der Stirnfläche 404 aufliegt, komprimiert wird und dadurch der Blutdurchfluss durch die entsprechenden Kapillaren 312 behindert werden kann (diskontinuierlich wird). In dem untersuchten bzw. beobachteten Bereich 600 des Gewebes 310, über dem sich die Vertiefung 408 befindet, kommt es nicht zu einer solchen Kompression und damit kann der Naturalzustand der Mikrozirkulation in den in diesem Bereich liegenden Kapillaren 312 beobachtet werden. Der Fluss in den Kapillaren 312 in dem beobachteten Bereich 600 ist nicht beeinträchtigt und damit kontinuierlich.

Im Allgemeinen kann in allen Ausführungsformen der Schutzkappe der Raum der Vertiefung 408 beispielsweise mit optisch transparenten, flüssigem Medium oder Luft gefüllt werden (mittels des Kanals 502). Hierdurch wird immer noch weniger Druck auf das Gewebe 310 ausgeübt als durch die anliegende Stirnfläche 404 der Schutzkappe. Durch die Möglichkeit einer variablen Befüllung der Vertiefung 408 mit beliebigen Medien ist auch denkbar, dass die Mikrozirkulation unter Einfluss einer variablen Druckstörung untersucht wird und so auf den Gesundheitszustand des Patienten geschlossen werden kann. Beispielsweise kann die Vertiefung 408 durch Zufuhr von Luft mit einem vordefinierten Druck beaufschlagt werden und nach Abbau dieses vordefinierten Drucks kann die Zeit gemessen werden, nach der die Mikrozirkulation wieder in ihren ursprünglichen Zustand zurückkehrt. Für die Durchführung dieser und weiterer Arten von dynamischen Messungen ist die vorliegende Schutzkappe bestens geeignet, da sie eine Schnittstelle zwischen dem Gewebe 310 und der Schutzkappe schafft, an welcher einerseits ein druckfreier Bereich (im Sinne von Kompression des Gewebes 312) bereitgestellt wird und andererseits zu Untersuchungszwecken durch gezieltes einleiten von Fluiden (z.B. Immersionsöl) oder Luft ein vordefinierter Druck auf das Gewebe 312 ausgeübt werden kann.

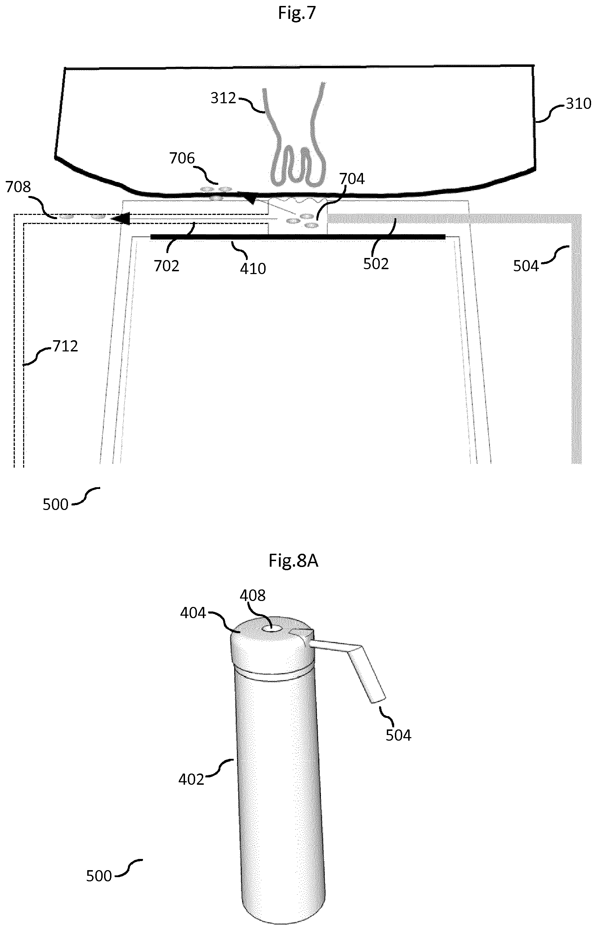

In **Figur 7** ist eine weitere Ausführungsform der Schutzkappe 500 dargestellt, wobei nur ihr vorderer Teil gezeigt ist. Die Schutzkappe 500 weist in diesem Ausführungsbeispiel zwei Kanäle auf, einen ersten Kanal 502 sowie einen zweiten Kanal 702, wobei beide Kanäle gleichartig sein können und von der Funktion her gegeneinander austausachbar sind. Ansonsten kann die in Figur 7 gezeigte Schutzkappe beispielsweise der in Figur 5 gezeigten Schutzkappe entsprechen.

Wie bereits erwähnt, behindern oft unerwünschtes Material 704 wie Detritus (u.a. Reste von roten Blutkörperchen), Speichel oder kleine Luftblasen die Sicht auf die Mikrozirkulation. Um diese Sichtbehinderungen zu beseitigen, kann mittels des ersten Kanals 502, welcher als Spülkanal verwendet werden kann, die Vertiefung 408 mit einer geeigneten Flüssigkeit gespült werden. Das unerwünschte Material 704 kann dann aus der Vertiefung 408 herausgespült werden und entlang der Berührungsstelle zwischen Gewebe 310 und Stirnfläche 404 der Schutzkappe 500 aus dem Beobachtungsbereich der Kamera wegtransportiert werden (erster Spülweg 706). Dazu kann es von Vorteil sein, wenn die Stirnfläche strukturiert ist und entsprechende Furchen oder Ähnliches aufweist, um den Abtransport des unerwünschten Materials zu erleichtern. Zusätzlich kann ein zweiter Kanal 702 (oder auch mehrere) vorgesehen sein, durch welchen die eingebrachte Flüssigkeit samt dem unerwünschten Material 704 aus der Vertiefung 408 abtransportiert werden kann (zweiter Spülweg 708). Wie in Figur 7 gezeigt, kann an den zweiten Kanal 702 optional eine Leitung 712 angeschlossen sein, über die der zweite Kanal 702 mit einem Sog beaufschlagt werden kann, um das unerwünschte Material 704 aus der Vertiefung 408 aktiv abzusaugen. Ansonsten kann der zweite Kanal 702 in der Mantelfläche 402 der Schutzkappe münden (d.h. ohne Verlängerungsleitung 712 zu einer Absaugpumpe) und dazu dienen, das unerwünschte Material aus der Vertiefung 408 herausfließen zu lassen.

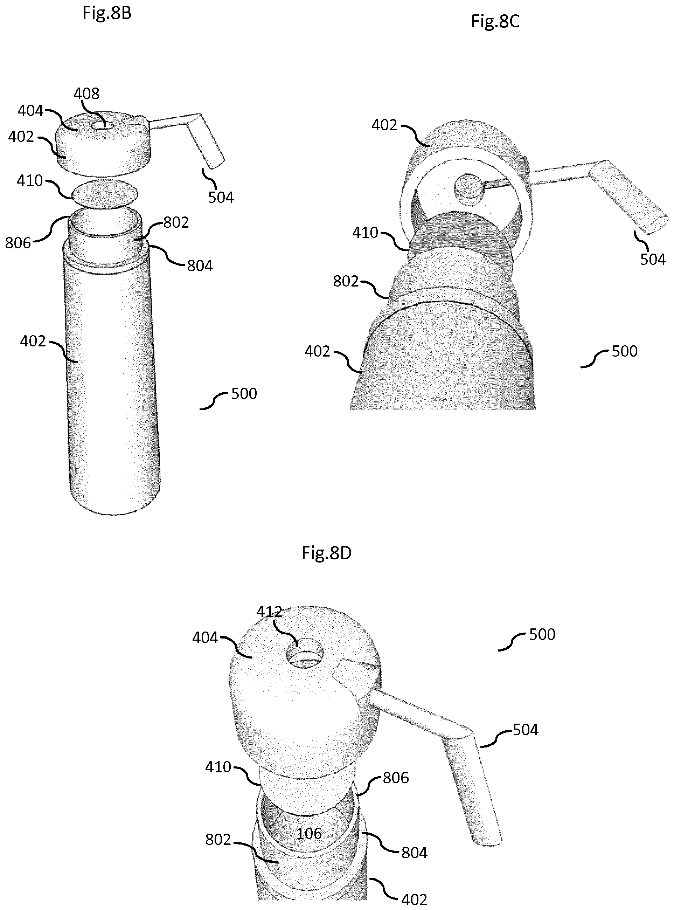

In den **Figuren 8A-8D** ist eine 3D-Dartellung der Schutzkappe gemäß weiteren Ausführungsbeispielen gezeigt, wobei Figur 8A eine räumliche Darstellung einer zusammengesetzten Form der Schutzkappe 500 zeigt, während Figuren 8B-8D Explosionsansichten aus verschiedenen Blickwinkeln zeigen. Die Bezugszeichen sind an den vorhergehenden Figuren orientiert, so dass bereits beschriebene Elemente der erfindungsgemäßen Kappe nicht erneut beschrieben werden.

Die in den Figuren 8A-8D gezeigte Ausführungsform zeichnet sich dadurch aus, dass die Spitze der Schutzkappe 500, im Wesentlichen also ein Teil der Schutzkappe 500, welcher die Stirnfläche 404 und einen daran angrenzenden Bereich der Mantelfläche 402 aufweist (nachfolgend als oberes Teil bezeichnet), vom Restteil, welcher den restlichen Teil der Mantelfläche 402 aufweist (nachfolgend als unteres Teil bezeichnet), lösbar ist. Bei einer derartigen Ausführungsform lässt sich der Boden 410 besonders einfach einsetzen oder ein bereits vorliegender Boden 410 wechseln. Wie dargestellt, kann der Boden 410 in den oberen Teil eingesetzt werden oder auf den Rand 806 eines erhöhten Steges 802 gelegt werden. Nach Zusammenstecken des oberen und unteren Teils wird durch Druck des erhöhten Steges 802 auf den Boden 410 dieser in seiner Lage fixiert. Dabei liegt das obere Teil mit seiner Unterkante auf einer Stufe 804 im unteren Teil an. In der dargestellten Ausführungsform liegt zwischen den beiden zusammensteckbaren Teilen eine Presspassung vor. Die Verbindung des oberen Teils mit dem unteren Teil kann jedoch genauso gut über eine Schaubverbindung realisiert werden.

## Patentansprüche

1. Schutzkappe für eine bildgebende Vorrichtung, aufweisend:
einen hohlen Schutzkappenkörper mit einer Stirnfläche, einer Mantelfläche und einem offenen hinteren Ende, durch welches die bildgebende Vorrichtung in die Schutzkappe eingesetzt werden kann;
eine Vertiefung, welche in der Stirnfläche angeordnet ist und einen Boden aufweist, welcher den innenliegenden Hohlraum gegenüber der Vertiefung dicht abschließt, wobei der Boden ein transparentes Material aufweist;
**dadurch gekennzeichnet, dass** die Schutzkappe weiterhin umfasst:
mindestens einen Kanal, welcher im vorderen Teil des Schutzkappenkörpers unter der Stirnfläche angeordnet ist;
wobei der mindestens eine Kanal eine erste Öffnung aufweist, welche in der Seitenwand der Vertiefung angeordnet ist, und eine zweite Öffnung aufweist, welche in der Mantelfläche des Schutzkappenkörpers angeordnet ist, und
wobei der mindestens eine Kanal eine Fluidverbindung zwischen der Vertiefung und der äußeren Umgebung der Schutzkappe bereitstellt.

2. Schutzkappe nach Anspruch 1,
wobei der Boden ein Deckglas aufweist; und
wobei ferner optional der Boden ein optisches Element aufweist.

3. Schutzkappe nach Anspruch 1 oder 2,
wobei an der Innenseite des Schutzkappenkörpers im Bereich der Stirnfläche mindestens ein Halteelement angeordnet ist, welches zur lösbaren Befestigung des Bodens der Vertiefung eingerichtet ist.

4. Schutzkappe nach einem der Ansprüche 1 bis 3,
wobei die Stirnfläche des Schutzkappenkörpers um die Vertiefung herum plan ausgebildet ist; oder
wobei die Stirnfläche des Schutzkappenkörpers als eine radial zur Vertiefung hin ansteigende oder abfallende Oberfläche ausgebildet ist.

5. Schutzkappe nach einem der Ansprüche 1 bis 4,
wobei die Stirnfläche des Schutzkappenkörpers zumindest in einem Bereich um die Vertiefung herum strukturiert ist.

6. Schutzkappe nach einem der Ansprüche 1 bis 5,
wobei in der Stirnfläche des Schutzkappenkörpers mindestens eine Furche angeordnet ist, welche sich zwischen der Vertiefung und dem äußeren Rand der Stirnfläche erstreckt.

7. Schutzkappe nach einem der Ansprüche 1 bis 6, ferner aufweisend:
mindestens einen weiteren Kanal, welcher im vorderen Teil des Schutzkappenkörpers unter der Stirnfläche verläuft,
wobei der mindestens eine weitere Kanal mindestens eine erste Öffnung aufweist, in der Stirnfläche angeordnet ist; und
wobei der mindestens eine weitere Kanal bevorzugt eine zweite Öffnung aufweist, welche in der Mantelfläche des Schutzkappenkörpers angeordnet ist.

8. Schutzkappe nach einem der Ansprüche 1 bis 7,
wobei die Seitenwand der Vertiefung und/oder die Stirnfläche der Schutzkappe ein lichtundurchlässiges Material aufweist.

## Claims

1. A protective cap for an imaging device, comprising:
a hollow protective cap body having a front surface, a shell surface and an open rear end through which the imaging device can be inserted into the protective cap;
a recess disposed in the front surface and having a bottom which seals the interior cavity from the recess bottom, wherein the bottom comprises a transparent material;
**characterized in that** the protective cap further comprises:
at least one channel disposed in the front portion of the protective cap body below the front surface;
wherein the at least one channel has a first opening disposed in the side wall of the recess and a second opening disposed in the shell surface of the protective cap body, and
wherein the at least one channel provides fluid communication between the recess and the outer environment of the protective cap.

2. Protective cap according to claim 1,
wherein the base comprises a cover glass; and
wherein further optionally the base comprises an optical element.

3. Protective cap according to claim 1 or 2,
wherein at least one retaining element is arranged on the inner side of the protective cap body in the region of the front surface, which retaining element is arranged for releasably securing the bottom of the recess.

4. Protective cap according to any one of claims 1 to 3,
wherein the front surface of the protective cap body is formed planar around the recess; or
wherein the front surface of the protective cap body is formed as a surface rising or falling radially towards the recess.

5. Protective cap according to any one of claims 1 to 4,
wherein the front surface of the protective cap body is structured at least in a region around the recess.

6. Protective cap according to any one of claims 1 to 5,
wherein at least one grove is arranged in the front surface of the protective cap body, which grove extends between the recess and the outer edge of the front surface.

7. Protective cap according to any one of claims 1 to 6, further comprising:
at least one further channel extending in the front part of the protective cap body below the front surface,
wherein the at least one further channel comprises at least one first opening, disposed in the front surface; and
wherein the at least one further channel preferably comprises a second opening, which is arranged in the shell surface of the protective cap body.

8. Protective cap according to any one of claims 1 to 7,
wherein the side wall of the recess and/or the front surface of the protective cap comprises an opaque material.

## Revendications

1. Capuchon protecteur pour un dispositif d'imagerie, comprenant :
un corps de capuchon protecteur creux avec une surface frontale, une surface d'enveloppe et une extrémité arrière ouverte à travers laquelle le dispositif d'imagerie peut être inséré dans le capuchon protecteur ;
un creux qui est disposé dans la surface frontale et qui présente un fond qui ferme de manière étanche l'espace creux situé à l'intérieur par rapport au creux, le fond présentant un matériau transparent ;
**caractérisé en ce que** le capuchon protecteur comprend en outre :
au moins un canal, qui est disposé dans la partie avant du corps du capuchon protecteur, sous la surface frontale ;
dans lequel ledit au moins un canal comprend une première ouverture disposée dans la paroi latérale du creux et une deuxième ouverture disposée dans la surface d'enveloppe du corps de capuchon protecteur, et
dans lequel ledit au moins un canal fournit une connexion fluidique entre le creux et l'environnement extérieur du capuchon protecteur.

2. Capuchon protecteur selon la revendication 1,
dans lequel le fond comprend un verre de couverture ; et
dans lequel le fond comprend en outre, facultativement, un élément optique.

3. Capuchon protecteur selon la revendication 1 ou 2,
dans lequel au moins un élément de retenue est disposé sur la face intérieure du corps de capuchon protecteur dans la zone de la surface frontale, lequel est conçu pour la fixation amovible du fond du creux.

4. Capuchon protecteur selon l'une quelconque des revendications 1 à 3,
dans lequel la surface frontale du corps du capuchon protecteur se présente sous une forme plane tout autour du creux ; ou
dans lequel la surface frontale du corps du capuchon protecteur est conçue comme une surface ascendante ou descendante radialement vers le creux.

5. Capuchon protecteur selon l'une quelconque des revendications 1 à 4, dans lequel la surface frontale du corps du capuchon protecteur est structurée au moins dans une zone autour du creux.

6. Capuchon protecteur selon l'une quelconque des revendications 1 à 5, dans lequel au moins un sillon est disposé dans la surface frontale du corps de capuchon protecteur, lequel s'étend entre le creux et le bord extérieur de la surface frontale.

7. Capuchon protecteur selon l'une quelconque des revendications 1 à 6, comprenant en outre :
au moins un autre canal qui s'étend dans la partie avant du corps du capuchon protecteur, sous la surface frontale,
dans lequel le, au moins un, autre canal présente au moins une première ouverture qui est disposée dans la surface frontale ; et
dans lequel le, au moins un, autre canal présente de préférence une deuxième ouverture qui est disposée dans la surface d'enveloppe du corps de capuchon protecteur.

8. Capuchon protecteur selon l'une quelconque des revendications 1 à 7, dans lequel la paroi latérale du creux et/ou la surface frontale du capuchon protecteur comprennent/comprend un matériau opaque.
